(19) Europäisches Patentamt
European Patent Office
Office européen des brevets

(11) **EP 4 418 188 A1**

(12) **EUROPEAN PATENT APPLICATION**
published in accordance with Art. 153(4) EPC

(43) Date of publication:
**21.08.2024 Bulletin 2024/34**

(21) Application number: **22881418.2**

(22) Date of filing: **14.10.2022**

(51) International Patent Classification (IPC):
*G06Q 30/02* (2023.01)    *G16H 40/20* (2018.01)
*G16H 50/70* (2018.01)    *G16H 10/60* (2018.01)
*G06Q 30/06* (2023.01)    *G06N 3/08* (2023.01)

(52) Cooperative Patent Classification (CPC):
**G06N 3/08; G06Q 30/02; G06Q 30/06;
G16H 10/60; G16H 40/20; G16H 50/70**

(86) International application number:
**PCT/KR2022/015637**

(87) International publication number:
**WO 2023/063791 (20.04.2023 Gazette 2023/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.10.2021 KR 20210136948
01.09.2022 KR 20220110826**

(71) Applicant: **Seoul National University Hospital
Seoul 03080 (KR)**

(72) Inventor: **KIM, Joonghee
Seongnam-si, Gyeonggi-do 13620 (KR)**

(74) Representative: **V.O.
P.O. Box 87930
2508 DH Den Haag (NL)**

(54) **CUSTOMIZED ADVERTISEMENT SYSTEM AND METHOD LINKED WITH MEDICAL INFORMATION ANALYSIS SERVICE**

(57)    A medical advertisement targeting system or apparatus according to an embodiment comprises: a reception unit which receives medical information including one or more attributes from one or more terminals; a first generation unit which generates summary information extracted from the medical information on the basis of the one or more attributes; a second generation unit which generates a summary vector corresponding to the summary information by digitizing the summary information using a pre-trained model; a calculation unit which calculates a degree of matching between the summary vector and one or more candidate produces by using a preset function; and a determination unit which determines a product-to-be-advertised to be displayed on a terminal-to-advertise, from among the candidate products, on the basis of the degree of matching. A medical advertisement targeting system, apparatus, and method according to an embodiment may provide more efficient targeted advertisements.

**FIG. 1**

**Description**

[Technical Field]

This application claims priority to all benefits arising from Korean Patent Application No. 10-2022-0110826 filed on September 1, 2022 and Korean Patent Application No. 10-2021-0136948 filed on October 14, 2021, the contents of which are incorporated herein by reference in their entirety.
Disclosed example embodiments are related to a technology for providing a customized advertisement linked with a medical information analysis service.

[Description of the Related Art]

The goal of an advertisement is to induce a user to purchase through information posted in the advertisement. However, general advertisements are randomly provided to an unspecified number of people without considering user needs.
In order to compensate for this problem, a customized advertisement system that considers the user's gender, age, region, usage history, and the like, has recently been developed. However, a customized advertisement system is required to precisely predict the user's consumption propensity. Therefore, there is a limitation that a customized advertisement system is only possible when the user's actions are collected, tracked, and analyzed.
In contrast, in the case of patients who have received medical services that inform them of their health status, etc., there is the difference that, regardless of the patient's consumption propensity, the medical services provided next have a high probability of reflecting diagnostic results derived from existing medical services, and multiple entities are involved in the purchase of medical products or services (this is also a characteristic of the medical healthcare industry). In other words, in the case of medical products or services, an important issue in order to provide more effective targeting advertisements is how to utilize information related to patient's medical information.

[Disclosure of the Invention]

[Technical Goals]

The disclosed example embodiments are intended to provide a customized advertising system and method linked with a medical information analysis service.

[Means for Solving the Task]

A medical advertisement targeting method according to an example embodiment is a method performed by a computing device including one or more processors, and a memory storing one or more programs executed by the one or more processors, the method including receiving medical information including one or more attributes from one or more terminals, generating summary information extracted from the medical information on the basis of the one or more attributes, generating a summary vector corresponding to the summary information using a pre-trained model, for example, by digitizing the summary information, calculating a degree of matching between the summary vector and one or more candidate products by using a preset function, and determining a product-to-be-advertised to be displayed on a terminal-to-advertise, from among the candidate products, on the basis of the degree of matching.
The attributes may include at least a part of one or more test item identifiers performed on a patient, one or more test type identifiers performed on a patient, medical device identifiers, medical device user identifiers, and patient identifiers.
The generating of the summary information may include generating test result summary information based on the test item identifier or test type identifier, respectively, based on the test item identifier or test type identifier, generating test result summary information based on the medical device identifier based on the medical device identifier, generating test result summary information based on the medical device user identifier based on the medical device user identifier, and/or generating test result summary information based on the patient identifier based on the patient identifier.
The summary information may be a numerical vector calculated by an encoder having an artificial neural network.
The calculating of the degree of matching further includes setting weights on the summary vector, setting the weight of the summary vector for the medical device identifier and/or the summary vector for the medical device user identifier higher than the weight of the summary vector for the patient identifier when the terminal-to-advertise is in a standby state and setting the weight of the summary vector for the patient identifier higher than the weight of the summary vector for the medical device identifier and the summary vector for the medical device user identifier when the transition of the terminal-to-advertise from a standby state to a use state occurs within a predetermined period of time from the time of setting the weights.

In the generating of the summary vector, the summary information for each identifier may be numerically distributed in a space of one or more dimensions using a pre-trained model, and a summary vector for each identifier may be generated based on the distributed numerical values.

In the generating of the summary vector, when there is only one summary information generated for each identifier, the summary information for each identifier may be numerically distributed in a space of one or more dimensions including a test item identifier axis using a pre-trained model, and a summary vector for the respective identifier may be generated based on the distributed numerical values, and when there is a plurality of summary information generated for each identifier, the summary information for each identifier may be numerically distributed in a space of two or more dimensions including a test item identifier axis and a time axis using a pre-trained model, and a single summary vector for each of the identifiers may be generated based on a mean value of the distributed numerical values.

The determining of the product-to-be-advertised may include giving priority to the candidate product based on the degree of matching, and determining a candidate product having the highest priority among the candidate products as the product-to-be-advertised.

The calculating of the degree of matching may include setting a first advertising weight for the summary vector based on a generation time of each medical information, and calculating the degree of matching by dot producting the first advertising weight with the summary vector.

The calculating of the degree of matching may include setting a second advertising weight proportional to an amount paid by an advertiser of each candidate product, and calculating the degree of matching by dot producting the second advertisement weight with the summary vector.

The calculating of the degree of matching may include setting a third advertising weight for a test item based on whether a matching ratio between a value of the test item included in a certain medical information and a value of the test item included in the rest of the medical information exceeds a predetermined threshold, and calculating the degree of matching by dot producting the third advertising weight with the summary vector.

The preset function may include an identity function, a step function, a Rectified Linear Unit function (ReLU), a sigmoid function, a K-means clustering algorithm, and/or a Support Vector Machine (SVM).

A method of providing data for medical product targeting advertisement according to an example embodiment includes receiving medical information including one or more attributes from one or more terminals, generating summary information extracted from the medical information on the basis of the one or more attributes, and generating a summary vector corresponding to the summary information by digitizing the summary information using a pre-trained model, wherein the attributes include at least a part of one or more test item identifiers performed on a patient, one or more test type identifiers performed on a patient, medical device identifiers, medical device user identifiers, and patient identifiers, wherein the generating of the summary information includes generating test result summary information based on the identifier based on the test item identifier or test type identifier, generating test result summary information based on the identifier based on the medical device identifier, generating test result summary information based on the identifier based on the medical device user identifier, and/or generating test result summary information based on the identifier based on the patient identifier.

A computer readable recording medium according to an example embodiment may record a program for executing the steps.

An apparatus 100 for medical advertisement targeting according to an example embodiment includes a reception unit which receives medical information including one or more attributes from one or more terminals, a first generation unit which generates summary information extracted from the medical information on the basis of the one or more attributes, a second generation unit which generates a summary vector corresponding to the summary information using a pre-trained model, for example, by digitizing the summary information, a calculation unit which calculates a degree of matching between the summary vector and candidate products by using a preset function, and a determination unit which determines a product-to-be-advertised to be displayed on a terminal-to-advertise, from among the candidate products, on the basis of the degree of matching.

The attributes may include at least a part of one or more test item identifiers performed on a patient, one or more test type identifiers performed on a patient, medical device identifiers, medical device user identifiers, and patient identifiers.

The first generation unit may generate test result summary information based on the test item identifier or test type identifier, respectively, based on at least the test item identifier or test type identifier, generate test result summary information based on the identifier based on the medical device identifier, generate test result summary information based on the identifier based on the medical device user identifier, and generate test result summary information based on the identifier based on the patient identifier.

The summary information may be a numerical vector calculated by an encoder having an artificial neural network.

The calculation unit may set weights on the summary vector, sets the weight of the summary vector for the medical device identifier and/or the summary vector for the medical device user identifier higher than the weight of the summary vector for the patient identifier when the terminal-to-advertise is in a standby state and sets the weight of the summary vector for the patient identifier higher than the weight of the summary vector for the medical device identifier and the summary vector for the medical device user identifier when the transition of the terminal-to-advertise from a standby state to a use state occurs within a predetermined period of time from the time of setting the weights.

The second generation unit may numerically distribute the summary information for each identifier in a space of one or more dimensions using a pre-trained model, and generate a summary vector for each identifier based on the distributed numerical values.

The second generation unit may, when there is only one summary information generated for each identifier, numerically distribute the summary information for each identifier in a space of one or more dimensions including a test item identifier axis using a pre-trained model, and generate a summary vector for the respective identifier based on the distributed numerical values, and when there is a plurality of summary information generated for each identifier, numerically distribute the summary information for each identifier in a space of two or more dimensions including a test item identifier axis and a time axis using a pre-trained model, and generate a single summary vector for each of the identifiers based on a mean value of the distributed numerical values.

The determination unit may give priority to the candidate product based on the degree of matching, and determine a candidate product having the highest priority among the candidate products as the product-to-be-advertised.

The calculation unit may set a first advertising weight for the summary vector based on a generation time of each medical information, and calculate the degree of matching by dot producting the first advertising weight with the summary vector.

The calculation unit may set a second advertising weight proportional to an amount paid by an advertiser of each candidate product, and calculate the degree of matching by dot producting the second advertisement weight with the summary vector.

The calculation unit may set a third advertising weight for a test item based on whether a matching ratio between a value of the test item included in a certain medical information and a value of the test item included in the rest of the medical information exceeds a predetermined threshold, and calculate the degree of matching by dot producting the third advertising weight with the summary vector.

The preset function may include an identity function, a step function, a Rectified Linear Unit function (ReLU), a sigmoid function, a K-means clustering algorithm, and/or a Support Vector Machine (SVM).

[Effects]

The disclosed example embodiments may provide a more effective customized advertisement service to patients who have a strong purchasing motivation based on information obtained from medical services, medical staff providing medical services, medical device manufacturers, pharmaceutical companies, and the like.

The disclosed example embodiments may provide a customized advertisement service based on various criteria by extracting summary information based on various criteria.

In the disclosed example embodiments, by setting different weights according to the use state of the terminal-to-advertise, it is possible to provide a more effective customized advertisement in consideration of the use state of the terminal-to-advertise.

The disclosed example embodiments may provide a customized advertisement in consideration of the latest medical information by setting a weight based on the generation time of the medical information.

In the disclosed example embodiments, by setting a weight proportional to the amount paid by the advertiser, a specific medical product may be more preferentially determined as a product-to-be-advertised.

[Brief Description of the Drawings]

FIG. 1 is a block diagram illustrating a medical advertisement targeting apparatus according to an example embodiment.

FIG. 2 is a flowchart illustrating a medical advertisement targeting method according to an example embodiment.

FIG. 3 is a block diagram illustrating and describing a computing environment including a computing device according to an example embodiment.

[Detailed Description for Carrying Out the Invention]

Hereinafter, a specific embodiment will be described with reference to the drawings.

The following detailed description is provided to aid in a comprehensive understanding of the methods, apparatus and/or systems described herein. However, this is illustrative only, and the present disclosure is not limited thereto.

In describing the embodiments, when it is determined that a detailed description of related known technologies related to the present disclosure may unnecessarily obscure the subject matter of the disclosed embodiments, a detailed description thereof will be omitted. In addition, terms to be described later are terms defined in consideration of functions in the present disclosure, which may vary according to the intention or custom of users or operators. Therefore, the definition should be made based on the contents throughout this specification. The terms used in the detailed description are only for describing embodiments, and should not be limiting. Unless explicitly used otherwise, expressions in the singular form include the meaning of the plural form. In this description, expressions such as "comprising" or "including" are intended to refer to certain components, numbers, steps, actions, elements, some or combination thereof, and it is not to be construed to exclude the presence or possibility of one or more other components, numbers, steps, actions, elements, parts or combinations thereof, other than those described.

Additionally, the embodiment described herein may have aspects of entirely hardware, partly hardware and partly software, or entirely software. The term "unit", "module", "device", "server" or "system" used herein refers to computer related entity such as hardware, software or a combination thereof. For example, the unit, module, device, server or system may refer to hardware that makes up a platform in part or in whole and/or software such as an application for operating the hardware.

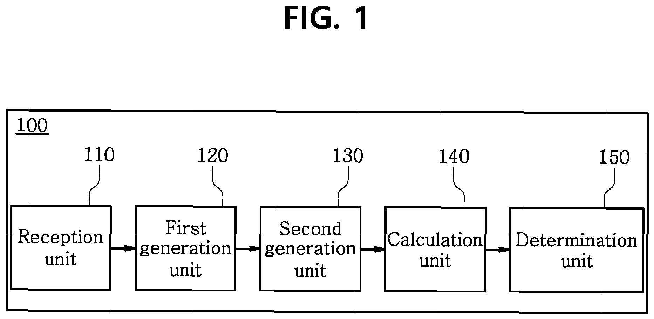

FIG. 1 is a block diagram illustrating a medical advertisement targeting apparatus 100 according to an example embodiment.

Referring to FIG. 1, the medical advertisement targeting apparatus 100 according to an example embodiment includes a reception unit 110, a first generation unit 120, a second generation unit 130, a calculation unit 140, and a determination unit 150.

The reception unit 110 receives medical information including one or more, for example, a plurality of, attributes from one or more terminals.

Here, the one or more terminals may include at least one of a medical device terminal, a medical device user terminal, and a patient terminal. A medical device terminal, a medical device user terminal, and a patient terminal may be one or more medical expert systems capable of processing one or more different input information or terminals capable of providing or receiving artificial intelligence services. For example, a medical device terminal, a medical device user terminal, and a patient terminal may be in the form of a personal computer, a laptop computer, a smart phone, a tablet PC, or a wearable device such as a smart band or a smart watch.

Medical information may be a patient's health condition provided through a medical expert system or artificial intelligence service. For example, medical information may include a diagnosis result for a patient. Specifically, medical information may have a value of 0 or more as a probability value for each diagnosis item, and each value for the diagnosis item may be normalized to be compatible. However, when there is no value for a certain identifier, a NULL value may be stored instead of the value for the certain identifier.

On the other hand, medical information has been described as information related to health conditions, but general information such as the patient's personal information including the patient's age and gender, the age, gender, and level of skill of the medical staff in charge, the medical department, and the registered use and frequency of use of the medical device, etc. may be further included. However, this is only exemplary and medical information is not limited to the above examples.

Medical information is an identifier of a terminal that generates, receives, or transmits medical information (ie, a medical device terminal, a medical device user terminal, and a patient terminal), an identifier of a test item related to a patient's health condition, an identifier of a test type, and a test equipment. At least a part of the identifier, the identifier of the medical device that measured the health condition of the patient, the identifier of the user (ie medical staff, etc.) using the medical device, and the identifier of the patient to be measured may be tagged and stored.

The medical information may be tagged and stored with at least a part of an identifier of a terminal that generates, receives, or transmits medical information (i.e., a medical device terminal, a medical device user terminal, and a patient terminal), an identifier of a test item related to the patient's health condition, an identifier of a test type, an identifier of a test equipment, an identifier of a medical device that measured the patient's health condition, an identifier of a user (i.e., a medical staff member, etc.) who used the medical device, and an identifier of the patient being measured.

For example, when medical information of a patient is received from a medical device terminal, the plurality of attributes may include an identifier of the medical device terminal. When the patient's medical information includes a certain value that is not 0 for a diabetes item, the plurality of attributes may include an identifier for the diabetes item as a test item identifier.

Medical information may be repeatedly measured from one patient, and may be measured once or repeatedly to several patients.

The first generation unit 120 generates summary information extracted from medical information on the basis of one or more attributes among a plurality of attributes.

Specifically, the first generation unit 120 may generate test result summary information based on at least the test item identifier or test type identifier. For example, when there is a high blood pressure item as a test item in the medical information, the first generation unit 120 may generate test result summary information based on an identifier for high blood pressure.

Also, the first generation unit 120 may generate test result summary information based on the identifier of the test equipment. For example, the first generation unit 120 may generate test result summary information based on an identifier of equipment classified and used for specific purposes, such as emergency rooms.

Also, the first generation unit 120 may generate test result summary information based on the identifier of the medical device user. For example, the first generation unit 120 may generate test result summary information based on an identifier of a medical staff in charge of a specific medical department.

Also, the first generation unit 120 may generate test result summary information based on the identifier of the patient. For example, the first generation unit 120 may generate test result summary information based on an identifier of a patient terminal having a history of a specific disease.

The second generation unit 130 generates a summary vector corresponding to the summary information by digitizing the summary information using a pre-trained model.

Specifically, the second generation unit 130 may numerically distribute the summary information for each identifier in a space of one or more dimensions using a pre-trained model, and generate a summary vector for each identifier based on the distributed numerical values.

For example, the second generation unit 130 may, when there is only one summary information generated for each identifier, numerically distribute the summary information for each identifier in a space of one or more dimensions including a test item identifier axis using a pre-trained model, and generate a summary vector for the respective identifier based on the distributed numerical values. When there is a plurality of summary information generated for each identifier, the second generation unit 130 may numerically distribute the summary information for each identifier in a space of two or more dimensions including a test item identifier axis and a time axis using a pre-trained model, and generate a single summary vector for each of the identifiers based on a mean value of the distributed numerical values.

Also, the second generation unit 130 may average a plurality of summary vectors generated for each identifier into one summary vector and regenerate it into a single summary vector.

The calculation unit 140 calculates a degree of matching between the summary vector and a plurality of candidate products by using a preset function.

Here, the preset function may include an identity function, a step function, a Rectified

Linear Unit function (ReLU), a sigmoid function, a K-means clustering algorithm, a Support Vector Machine (SVM), and/or a deep learning model designed to predict advertising effectiveness in consideration of the number of clicks, expected revenue, etc., but is not limited thereto..

The preset function may be defined by Equation 1 below.

[Equation 1]

$$F_{item}(x) = a_1 f_1(x_1) + a_2 f_2(x_2) + a_3 f_3(x_3) + \cdots + a_n f_n(x_n) + e$$

wherein $F_{item}(x)$ is the preset function, $x$ is a summary vector with a size of $n$, $x_n$ is the $n^{th}$ element of $x$, $a_1$ to $a_n$ are random real numbers, and $e$ is a random number.

Meanwhile, the preset function may be a function set by an advertising company or an advertiser.

The calculation unit 140 may set weights on the summary vector.

For example, when the terminal-to-advertise is in a standby state, the calculation unit 140 may set the weight in the summary vector for the medical device identifier and/or the summary vector for the medical device user identifier to be higher than the weight set in the summary vector for the patient identifier.

For example, when the transition of the terminal-to-advertise from a standby state to a use state occurs within a predetermined period of time from the time of setting the weights, the calculation unit 140 may set the weight set in the summary vector for the patient identifier to be higher than the weight set in the summary vector for the medical device identifier and the summary vector for the medical device user identifier.

As another example, the calculation unit 140 may set a first advertising weight for the summary vector based on a generation time of each medical information, and calculate the degree of matching by dot producting the first advertising weight with the summary vector.

As another example, the calculation unit 140 may set a second advertising weight proportional to an amount paid by an advertiser of each candidate product, and calculate the degree of matching by dot producting the second advertisement weight with the summary vector.

As another example, the calculation unit 140 may set a third advertising weight for a test item based on whether a matching ratio between a value of the test item included in a certain medical information and a value of the test item included in the rest of the medical information exceeds a predetermined threshold, and calculate the degree of matching by dot producting the third advertising weight with the summary vector.

The determination unit 150 determines a product-to-be-advertised to be displayed on a terminal-to-advertise, from among one or more, for example, a plurality of, candidate products on the basis of the degree of matching.

Here, the determination unit 150 may determine a product-to-be-advertised to be displayed on at least one of a medical device terminal, a medical device user terminal, and a patient terminal, and there may be one or more products-to-be-advertised.

Meanwhile, although it has been described that the terminals-to-advertise are medical device terminals, medical device user terminals, and patient terminals, they are not necessarily limited thereto, and devices capable of displaying advertisements are interpreted to be included therein.

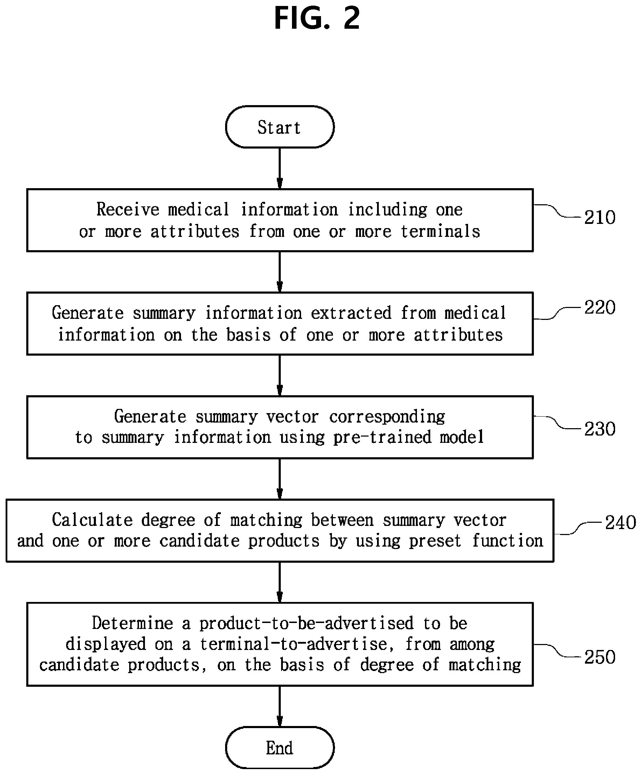

FIG. 2 is a flowchart illustrating a medical advertisement targeting method according to an example embodiment. The method shown in FIG. 2 may be performed by, for example, the medical advertisement targeting apparatus 100 described above.

Referring to FIG. 2, first, in step 210, the medical advertisement targeting apparatus 100 receives medical information including one or more, for example, a plurality of, attributes from one or more terminals.

Next, in step 220, the medical advertisement targeting apparatus 100 generates summary information extracted from the medical information on the basis of one or more attributes among the plurality of attributes.

Next, in step 230, the medical advertisement targeting apparatus 100 generates a summary vector corresponding to the summary information by digitizing the summary information using a pre-trained model.

Next, in step 240, the medical advertisement targeting apparatus 100 calculates a degree of matching between the summary vector and one or more, for example, a plurality of, candidate products by using a preset function.

Next, in step 250, the medical advertisement targeting apparatus 100 determines a product-to-be-advertised to be displayed on a terminal-to-advertise, from among the one or more, for example, a plurality of, candidate products on the basis of the degree of matching.

FIG. 2 shown above has been described with reference to the order presented in the drawing. For the sake of explanation, the method is illustrated and described as a series of blocks, but the present disclosure is not limited to the order of the blocks, and some blocks may occur in a different order or concurrently with other blocks than shown and described herein, and various other branches, flow paths, and sequences of blocks that achieve the same or similar results may be implemented. Also, not all blocks shown may be required for implementation of the methods described herein.

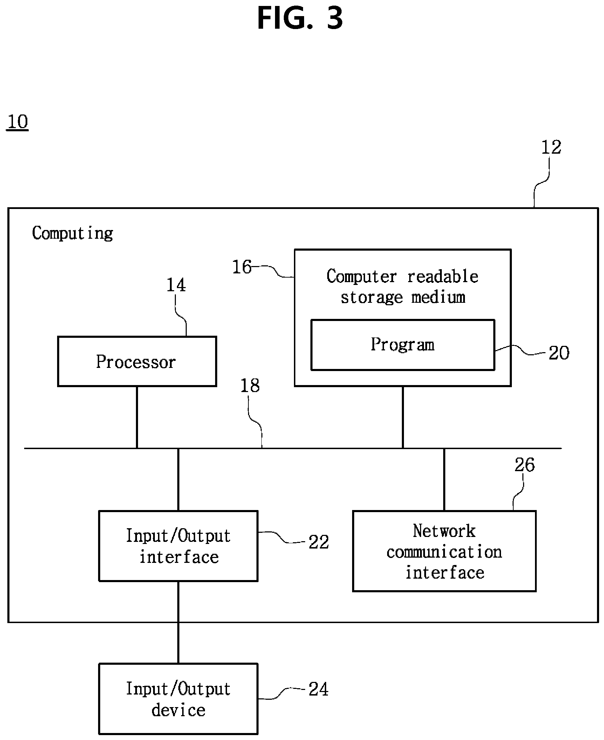

FIG. 3 is a block diagram illustrating and describing a computing environment including a medical advertisement targeting apparatus 100 according to an example embodiment.

The communication bus 18 connects a variety of different components of the computing device 12, including the processor 14 and the computer-readable storage medium 16.

The computing device 12 may also include one or more input/output interfaces 22 providing interfaces for one or more input/output devices 24 and one or more network communication interfaces 26. The input/output interfaces 22 and the network communication interfaces 26 are connected to the communication bus 18. The input/output devices 24 may be connected to other components of the computing device 12 through the input/output interfaces 22. The illustrative input/output devices 24 may include input devices, such as a pointing device (e.g., a mouse or a track pad), a keyboard, a touch input device (e.g., a touch pad or a touchscreen), a voice or audio input device, various types of sensor devices, and/or an image capturing device, and/or output devices, such as a display device, a printer, a speaker, and/or a network card. The illustrative input/output devices 24 may be included inside the computing device 12 as components of the computing device 12 or be connected to the computing device 12 as separate devices distinct from the computing device 12.

The embodiments of the present disclosure may include a program for running the methods described herein on a computer and a computer-readable recording medium including the program. The computer-readable recording medium may include program instructions, local data files, local data structures, and the like, alone or in a combination thereof. The medium may be specially designed and configured for the present disclosure or may be known to and used by a person having ordinary skill in the field of computer software. Examples of the computer-readable recording medium may include magnetic media, such as a hard disk, a floppy disk, and a magnetic tape, optical media, such as a compact disc read-only memory (CD-ROM) and a digital versatile disc (DVD), and hardware devices specially configured to store and perform program instructions, such as a ROM, a RAM, and a flash memory. Examples of the program instructions may include machine language code made by a compiler and a high-level language code executable by a computer using an interpreter or the like.

While the exemplary embodiments of the present disclosure have been described in detail hereinabove, a person having ordinary knowledge in the technical field to which the present disclosure pertains will appreciate that various modifications are possible to the foregoing embodiments without departing from the scope of the present disclosure. Therefore, the scope of protection of the present disclosure shall not be limited to the foregoing embodiments but shall be defined by the appended Claims and equivalents thereof.

[Industrial Applicability]

A medical advertisement targeting system, apparatus, and method according to an example embodiment may be used in the advertisement industry for customized recommendation of various medical products or medical services including drugs or medical devices.

**Claims**

1. A medical advertisement targeting method performed by a computing device comprising one or more processors; and a memory storing one or more programs executed by the one or more processors, the method comprising:

   receiving medical information including one or more attributes from one or more terminals;
   generating summary information extracted from the medical information on the basis of the one or more attributes;
   generating a summary vector corresponding to the summary information using a pre-trained model;
   calculating a degree of matching between the summary vector and one or more candidate products by using a preset function; and
   determining a product-to-be-advertised to be displayed on a terminal-to-advertise, from among the candidate products, on the basis of the degree of matching.

2. The method of claim 1, wherein the attributes include at least a part of one or more test item identifiers performed on a patient, one or more test type identifiers performed on a patient, medical device identifiers, medical device user identifiers, and patient identifiers.

3. The method of claim 2, wherein the generating of the summary information comprises:

   generating test result summary information based on the test item identifier or test type identifier, respectively, based on the test item identifier or test type identifier;
   generating test result summary information based on the medical device identifier based on the medical device identifier;
   generating test result summary information based on the medical device user identifier based on the medical device user identifier; and/or
   generating test result summary information based on the patient identifier based on the patient identifier.

4. The method of claim 2, wherein the summary information is a numerical vector calculated by an encoder having an artificial neural network.

5. The method of claim 2, wherein the calculating of the degree of matching further comprises setting weights on the summary vector,

   setting the weight of the summary vector for the medical device identifier and/or the summary vector for the medical device user identifier higher than the weight of the summary vector for the patient identifier when the terminal-to-advertise is in a standby state and
   setting the weight of the summary vector for the patient identifier higher than the weight of the summary vector for the medical device identifier and the summary vector for the medical device user identifier when the transition of the terminal-to-advertise from a standby state to a use state occurs within a predetermined period of time from the time of setting the weights.

6. The method of claim 3, wherein in the generating of the summary vector, the summary information for each identifier is numerically distributed in a space of one or more dimensions using a pre-trained model, and a summary vector for each identifier is generated based on the distributed numerical values.

7. The method of claim 6, wherein in the generating of the summary vector, when there is only one summary information generated for each identifier, the summary information for each identifier is numerically distributed in a space of one or more dimensions including a test item identifier axis using a pre-trained model, and a summary vector for the respective identifier is generated based on the distributed numerical values, and

wherein when there is a plurality of summary information generated for each identifier, the summary information for each identifier is numerically distributed in a space of two or more dimensions including a test item identifier axis and a time axis using a pre-trained model, and a single summary vector for each of the identifiers is generated based on a mean value of the distributed numerical values.

8. The method of claim 1, wherein the determining of the product-to-be-advertised comprises giving priority to the candidate product based on the degree of matching; and
determining a candidate product having the highest priority among the candidate products as the product-to-be-advertised.

9. The method of claim 1, wherein the calculating of the degree of matching comprises:

setting a first advertising weight for the summary vector based on a generation time of each medical information; and
calculating the degree of matching by dot producting the first advertising weight with the summary vector.

10. The method of claim 1, wherein the calculating of the degree of matching comprises:

setting a second advertising weight proportional to an amount paid by an advertiser of each candidate product; and
calculating the degree of matching by dot producting the second advertisement weight with the summary vector.

11. The method of claim 1, wherein the calculating of the degree of matching comprises setting a third advertising weight for a test item based on whether a matching ratio between a value of the test item included in a certain medical information and a value of the test item included in the rest of the medical information exceeds a predetermined threshold; and
calculating the degree of matching by dot producting the third advertising weight with the summary vector.

12. The method of claim 1, wherein the preset function includes an identity function, a step function, a Rectified Linear Unit function (ReLU), a sigmoid function, a K-means clustering algorithm, and/or a Support Vector Machine (SVM).

13. A method of providing data for medical product targeting advertisement, comprising:

receiving medical information including one or more attributes from one or more terminals;
generating summary information extracted from the medical information on the basis of the one or more attributes; and
generating a summary vector corresponding to the summary information using a pre-trained model,
wherein the attributes include at least a part of one or more test item identifiers performed on a patient, one or more test type identifiers performed on a patient, medical device identifiers, medical device user identifiers, and patient identifiers, and
wherein the generating of the summary information comprises:

generating test result summary information based on the test item identifier or test type identifier;
generating test result summary information based on the medical device identifier;
generating test result summary information based on the medical device user identifier; and
generating test result summary information based on the patient identifier.

14. A computer readable recording medium for executing the method according to any one of claims 1 to 13.

15. An apparatus for medical advertisement targeting comprising:

a reception unit which receives medical information including one or more attributes from one or more terminals;
a first generation unit which generates summary information extracted from the medical information on the basis of the one or more attributes;
a second generation unit which generates a summary vector corresponding to the summary information using a pre-trained model;
a calculation unit which calculates a degree of matching between the summary vector and one or more candidate products by using a preset function; and

a determination unit which determines a product-to-be-advertised to be displayed on a terminal-to-advertise, from among the candidate products, on the basis of the degree of matching.

16. The apparatus of claim 15, wherein the attributes include at least a part of one or more test item identifiers performed on a patient, one or more test type identifiers performed on a patient, medical device identifiers, medical device user identifiers, and patient identifiers.

17. The apparatus of claim 16, wherein the first generation unit generates test result summary information based on at least the test item identifier,

    generates test result summary information based on the test type identifier, generates test result summary information based on the medical device identifier, generates test result summary information based on the medical device user identifier, and
    generates test result summary information based on the patient identifier.

18. The apparatus of claim 16, wherein the summary information is a numerical vector calculated by an encoder having an artificial neural network.

19. The apparatus of claim 16, wherein the calculation unit sets weights on the summary vector,

    sets the weight of the summary vector for the medical device identifier and/or the summary vector for the medical device user identifier higher than the weight of the summary vector for the patient identifier when the terminal-to-advertise is in a standby state and
    sets the weight of the summary vector for the patient identifier higher than the weight of the summary vector for the medical device identifier and the summary vector for the medical device user identifier when the transition of the terminal-to-advertise from a standby state to a use state occurs within a predetermined period of time from the time of setting the weights.

20. The apparatus of claim 17, wherein the second generation unit numerically distributes the summary information for each identifier in a space of one or more dimensions using a pre-trained model, and generates a summary vector for each identifier based on the distributed numerical values.

21. The apparatus of claim 20, wherein the second generation unit, when there is only one summary information generated for each identifier, numerically distributes the summary information for each identifier in a space of one or more dimensions including a test item identifier axis using a pre-trained model, and generates a summary vector for the respective identifier based on the distributed numerical values, and
    when there is a plurality of summary information generated for each identifier, numerically distributes the summary information for each identifier in a space of two or more dimensions including a test item identifier axis and a time axis using a pre-trained model, and generates a single summary vector for each of the identifiers based on a mean value of the distributed numerical values.

22. The apparatus of claim 15, wherein the determination unit gives priority to the candidate product based on the degree of matching, and determines a candidate product having the highest priority among the candidate products as the product-to-be-advertised.

23. The apparatus of claim 15, wherein the calculation unit sets a first advertising weight for the summary vector based on a generation time of each medical information, and calculates the degree of matching by dot producting the first advertising weight with the summary vector.

24. The apparatus of claim 15, wherein the calculation unit sets a second advertising weight proportional to an amount paid by an advertiser of each candidate product, and calculates the degree of matching by dot producting the second advertisement weight with the summary vector.

25. The apparatus of claim 15, wherein the calculation unit sets a third advertising weight for a test item based on whether a matching ratio between a value of the test item included in a certain medical information and a value of the test item included in the rest of the medical information exceeds a predetermined threshold, and calculates the degree of matching by dot producting the third advertising weight with the summary vector.

26. The apparatus of claim 15, wherein the preset function includes an identity function, a step function, a Rectified Linear Unit function (ReLU), a sigmoid function, a K-means clustering algorithm, and/or a Support Vector Machine (SVM).

# FIG. 1

# FIG. 2

Start

Receive medical information including one
or more attributes from one or more terminals — 210

Generate summary information extracted from medical
information on the basis of one or more attributes — 220

Generate summary vector corresponding
to summary information using pre-trained model — 230

Calculate degree of matching between summary vector
and one or more candidate products by using preset function — 240

Determine a product-to-be-advertised to be
displayed on a terminal-to-advertise, from among
candidate products, on the basis of degree of matching — 250

End

# FIG. 3

10

12

Computing

16

Computer readable
storage medium

14

Processor

Program

20

18

Input/Output
interface

22

26

Network
communication
interface

Input/Output
device

24

| | INTERNATIONAL SEARCH REPORT | International application No. |
|---|---|---|
| | | **PCT/KR2022/015637** |

**A. CLASSIFICATION OF SUBJECT MATTER**

**G06Q 30/02**(2012.01)i; **G16H 40/20**(2018.01)i; **G16H 50/70**(2018.01)i; **G16H 10/60**(2018.01)i; **G06Q 30/06**(2012.01)i; **G06N 3/08**(2006.01)i

According to International Patent Classification (IPC) or to both national classification and IPC

**B. FIELDS SEARCHED**

Minimum documentation searched (classification system followed by classification symbols)

G06Q 30/02(2012.01); G06Q 10/02(2012.01); G06Q 30/00(2006.01); G06Q 50/00(2006.01); G06Q 50/22(2012.01); G06Q 50/30(2012.01); G16H 20/10(2018.01); G16H 20/70(2018.01); G16H 50/70(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 의료(medical), 정보(information), 추출(extract), 요약(summary), 벡터(vector), 매칭(matching), 광고(advertisement), 추천(recommend), 상품(product)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2018-0016026 A (LEE, Beong Chul) 14 February 2018 (2018-02-14) See paragraphs [0010], [0017]-[0025] and [0033]; and figures 1-4. | 1-4,6-8,12-18,20-22,26 |
| A | | 5,9-11,19,23-25 |
| Y | KR 10-1968200 B1 (CHOI, Jeong Min) 12 April 2019 (2019-04-12) See paragraphs [0001], [0025]-[0026], [0036]-[0039], [0048]-[0054] and [0146]; and figures 1-4. | 1-4,6-8,12-18,20-22,26 |
| A | KR 10-2020-0049606 A (KOREA UNIVERSITY RESEARCH AND BUSINESS FOUNDATION) 08 May 2020 (2020-05-08) See paragraphs [0011]-[0012], [0034] and [0045]; and figure 6. | 1-26 |
| A | KR 10-1788030 B1 (KAII COMPANY INC.) 15 November 2017 (2017-11-15) See paragraphs [0005], [0066] and [0138]; and figures 1 and 5-7. | 1-26 |

| ✓ Further documents are listed in the continuation of Box C. | ✓ See patent family annex. |
|---|---|

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 February 2023** | **03 February 2023** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2022/015637**

**C.** DOCUMENTS CONSIDERED TO BE RELEVANT

| Category* | Citation of document, with indication, where appropriate, of the relevant passages | Relevant to claim No. |
|---|---|---|
| A | US 2011-0046970 A1 (FONTENOT, Craig) 24 February 2011 (2011-02-24) See paragraphs [0038], [0069] and [0088]; and figures 1-2. | 1-26 |

Form PCT/ISA/210 (second sheet) (July 2022)

**INTERNATIONAL SEARCH REPORT**
Information on patent family members

| International application No. |
| --- |
| **PCT/KR2022/015637** |

| Patent document cited in search report | | | Publication date (day/month/year) | Patent family member(s) | | | Publication date (day/month/year) |
| --- | --- | --- | --- | --- | --- | --- | --- |
| KR | 10-2018-0016026 | A | 14 February 2018 | None | | | |
| KR | 10-1968200 | B1 | 12 April 2019 | None | | | |
| KR | 10-2020-0049606 | A | 08 May 2020 | WO | 2020-091375 | A2 | 07 May 2020 |
| KR | 10-1788030 | B1 | 15 November 2017 | None | | | |
| US | 2011-0046970 | A1 | 24 February 2011 | US | 2007-0299910 | A1 | 27 December 2007 |
| | | | | WO | 2010-091438 | A1 | 12 August 2010 |

Form PCT/ISA/210 (patent family annex) (July 2022)

**EP 4 418 188 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220110826
- KR 1020210136948